# EUROPÄISCHE PATENTSCHRIFT

(11) **EP 3 076 882 B1**
(45) Veröffentlichungstag und Bekanntmachung des Hinweises auf die Patenterteilung: **28.08.2019**
(21) Anmeldenummer: 14833337.0
(22) Anmeldetag: 10.11.2014
(51) Int. Cl.: A61B 17/29, A61B 17/00, A61B 34/30, A61B 34/00

(54) **INSTRUMENT, INSBESONDERE EIN MEDIZINISCH-ENDOSKOPISCHES INSTRUMENT ODER TECHNOSKOP**
INSTRUMENT, IN PARTICULAR A MEDICAL ENDOSCOPIC INSTRUMENT OR TECHNOSCOPE
INSTRUMENT, EN PARTICULIER INSTRUMENT MÉDICAL-ENDOSCOPIQUE OU TECHNOSCOPE

(30) Priorität: 03.12.2013 DE 102013224753
(43) Veröffentlichungstag der Anmeldung: 12.10.2016
(73) Patentinhaber: Richard Wolf GmbH, 75438 Knittlingen (DE)
(72) Erfinder: TEICHTMANN, Elmar, 75015 Bretten (DE)
(74) Vertreter: Patentanwälte Vollmann Hemmer Lindfeld Partnerschaft mbB
(86) Internationale Anmeldenummer: PCT/DE2014/200624
(87) Internationale Veröffentlichungsnummer: WO 2015/081946

(56) Entgegenhaltungen:
- EP-A2- 1 886 630
- EP-A2- 2 014 252
- WO-A1-2009/079781
- JP-U- S6 310 088
- US-A- 5 710 870
- US-A1- 2007 023 477

## Beschreibung

Die Erfindung betrifft ein Schaftinstrument und insbesondere ein medizinisch-endoskopisches Schaftinstrument oder ein Technoskop.

Im Bereich der Medizin und dort insbesondere im Bereich der Endoskopie werden Schaftinstrumente eingesetzt, die an dem distalen Ende eines Schaftes einen Instrumentenkopf mit einem daran angeordneten Werkzeug aufweisen. Instrumente dieser Art, bei denen es sich zum Beispiel um Fass- oder Schneidinstrumente handeln kann, finden darüber hinaus auch auf anderen Gebieten, beispielsweise bei Technoskopen zum Einsatz in Hohlräumen technischer Objekte Verwendung.

Den Ausgangspunkt der Erfindung bilden solche Instrumente, bei denen der Instrumentenkopf relativ zu dem Schaft und das Werkzeug oder zumindest ein Teil des Werkzeugs relativ zu einem Werkzeugträger des Instrumentenkopfes abwinkelbar sind. Ein derartiges Instrument ist aus US 2007/0208375 A1 bekannt. Dieses Instrument weist einen distalseitig eines Schaftes angeordneten Instrumentenkopf auf, der einen Werkzeugträger umfasst, welcher über ein längliches Gelenkteil gelenkig an dem distalen Ende des Schaftes angelenkt ist. Der Werkzeugträger ist in der Abwinkelungsebene des Gelenkteils abwinkelbar an dem distalen Ende des Gelenkteils angelenkt. Zwecks Gewährleistung einer kontrollierten Abwinkelung des Werkzeugträgers relativ zu dem Schaft und dem Gelenkteil ist der Werkzeugträger mit dem Schaft über eine Wälzkörperpaarung gekoppelt. Zur Steuerung der Abwinkelung des Instrumentenkopfes dienen Seilzüge, welche direkt auf das Gelenkteil wirken. Hierbei erweist es sich als nachteilig, dass zur Steuerung der Abwinkelung des Instrumentenkopfes konstruktionsbedingt nur vergleichsweise kleine Momente auf das Gelenkteil übertragen werden können. Ein weiterer Nachteil dieses Instruments besteht darin, dass das Gelenkteil selbst bei einer Gesamtabwinkelung des Instrumentenkopfes von +/-120° nur eine seitliche Auslenkung von +/- 60° erfährt, sodass die Abwinkelung des Instrumentenkopfes relativ zu dem Schaft nur verhältnismäßig grob einstellbar ist.

Aus JP S-6310088 U ist ein endoskopisches Instrument bekannt, bei welchen ein am Ende des Schaftes angeordneter Instrumentenkopf über ein Gelenkteil gegenüber dem Schaft abwinkelbar ist, wobei distalseitig an dem Gelenkteil ein demgegenüber abwinkelbar angelenkter Werkzeugträger vorgesehen ist. Am proximalen Ende des Gelenkteils ist eine Verzahnung ausgebildet, welche mit einem in dem Schaft drehbar gelagerten und mit einem Seilzugpart zur Bewegungssteuerung des Instrumentenkopfes bewegungsgekoppelten Verzahnkörper in Eingriff ist. Ein vergleichbares Schaftinstrument ist aus EP 1 886 630 A2 bekannt.

Ausgehend von diesem Stand der Technik liegt der Erfindung die Aufgabe zugrunde, ein Schaftinstrument der in Rede stehenden Art zu verbessern.

Diese Aufgabe wird durch ein Schaftinstrument mit den in Anspruch 1 angegebenen Merkmalen gelöst. Vorteilhafte Weiterbildungen dieses Schaftinstruments ergeben sich aus den Unteransprüchen.

Das erfindungsgemäße Schaftinstrument ist bevorzugt ein medizinisch-endoskopisches Instrument. Es kann sich bei ihm aber auch um ein Technoskop handeln, das in schwer zugänglichen Hohlräumen technischer Objekte eingesetzt wird. Wie bei solchen Schaftinstrumenten üblich, weist auch das erfindungsgemäße Schaftinstrument einen länglichen, vorzugsweise gerade und starr ausgebildeten Schaft mit einem distalseitig daran angeordneten Instrumentenkopf auf.

Der Instrumentenkopf weist mindestens ein an dem distalen Schaftende abwinkelbar angelenktes Gelenkteil und einen distalseitig an dem Gelenkteil in der Abwinkelungsebene des Gelenkteils abwinkelbar angelenkten Werkzeugträger auf. Dabei ist an dem proximalen Ende des Gelenkteils eine Verzahnung vorzugsweise in Form eines Zahnradsegments ausgebildet, die mit einem in dem Schaft drehbar gelagerten und mit einem Seilzugpaar zur Bewegungssteuerung des Instrumentenkopfes bewegungsgekoppelten Verzahnkörper in Eingriff ist. Die Abwinkelung des Instrumentenkopfes erfolgt hierbei durch eine Drehbewegung des Verzahnkörpers, die durch Zugbeanspruchung eines der Seilzüge des mit dem Verzahnkörper bewegungsgekoppelten antagonistisch auf den Verzahnkörper wirkenden Seilzugpaares verursacht wird. Aufgrund der Maßnahme, das Gelenkteil und damit den Werkzeugträger bzw. den gesamten Instrumentenkopf über den in dem Schaft proximalseitig der Anlenkung des Gelenkteils angeordneten Verzahnkörper zu verschwenken, ist es bei dem erfindungsgemäßen Schaftinstrument nicht erforderlich, die Seilzüge über den Abwinkelungsbereich zwischen Schaft und Instrumentenkopf hinweg zu führen. Stattdessen sind die Seilzüge unabhängig von der Abwinkelung des Instrumentenkopfes in dem Schaft immer weitestgehend linear geführt, sodass bei der Abwinkelung des Instrumentenkopfes einer der Seilzüge in gleichem Maße in distaler Richtung bewegt wird, wie der andere in proximaler Richtung bewegt wird.

Der Verzahnkörper ist erfindungsgemäß quer zu seiner Drehachse zweigeteilt ausgebildet, wobei die beiden Teile des Verzahnkörpers relativ zueinander drehbar sind. In Verbindung mit dieser Ausgestaltung ist weiter erfindungsgemäß vorgesehen, dass jeder der beiden Teile des Verzahnkörpers drehfest mit einer Betätigungsrolle verbunden ist, an welcher jeweils ein Seilzug bezogen auf den anderen Seilzug antagonistisch angreift. Diese konstruktiven Maßnahmen dienen dazu, ein bei einem einteiligen Verzahnkörper bei Umkehr der Drehrichtung des Verzahnkörpers ggf. auftretendes Zahnspiel zu verhindern, da aufgrund der Vorspannung der mit den beiden Betätigungsrollen verbundenen Seilzüge zumindest immer eines der Teile des Verzahnkörpers mit der an dem proximalen Ende des Gelenkteils ausgebildeten Verzahnung spielfrei in Eingriff ist.

Aus WO 2009/079781 A1 zählt es bereits zum Stand der Technik, ein Stirnrad quer zu seiner Drehachse geteilt auszubilden und dazwischen eine Feder anzuordnen, welche dafür sorgt, dass unabhängig von der Drehrichtung des Zahnrades stets eine Flanke im Eingriff ist und somit das Zahnrad spielfrei mit dem Gegenrad kämmt. Die dort dargestellte Ausführung betrifft einen Manipulator, also eine Maschine zur Steuerung eines medizinischen Operationsinstrumentes.

Zur Bewegungskopplung des Seilzugpaares mit dem Verzahnkörper sind zwei Betätigungsrollen vorgesehen, die achsgleich
mit dem Verzahnkörper in dem Schaft drehbar gelagert ist und mit dem Verzahnkörper drehfest verbunden sind. Mit diesen Betätigungsrollen ist das Seilzugpaar zweckmäßigerweise derart verbunden, dass die Betätigungsrolle und der Verzahnkörper bei einer Zugbeanspruchung eines ersten Seilzugs des Seilzugpaares in eine erste Richtung gedreht werden und bei einer Zugbeanspruchung des anderen Seilzugs des Seilzugpaares in eine zweite, entgegengesetzte Richtung gedreht werden. Die Befestigung der Seilzüge des Seilzugpaares an den Befestigungsrollen erfolgt günstig umfänglich der Befestigungsrolle, wobei die Seilzüge an den Betätigungsrollen typischerweise bezogen auf die Drehachse der Betätigungsrollen an zwei einander gegenüberliegenden Seiten befestigt sind. Als Betätigungsrolle ist zwar bevorzugt ein Bauteil mit einer kreisförmigen Querschnittskontur vorgesehen, allerdings sind im Sinne der Erfindung unter dem Begriff "Betätigungsrolle" grundsätzlich alle Bauteile zu verstehen, die lediglich einen teilkreisförmigen oder teilkreisähnlichen Umfangsabschnitt aufweisen, der von den beiden Seilzügen teilweise umschlungen wird, ansonsten aber eine beliebige Umfangskontur aufweisen können.

Sinnvollerweise ist vorzusehen, dass die Betätigungsrolle einen möglichst großen Wirkradius aufweist, um bei möglichst geringem Kraftaufwand das zum Abwinkeln des Instrumentenkopfes erforderliche Bewegungsmoment erzeugen zu können. Zu diesem Zweck weist die in dem Schaft angeordnete Betätigungsrolle vorteilhaft einen Durchmesser auf, der im Wesentlichen mit dem Innendurchmesser des Schaftes übereinstimmt. Diese Dimensionierung der Betätigungsrolle setzt typischerweise voraus, dass die Betätigungsrolle zumindest in unmittelbarer Nähe der Mittelachse des Schaftes drehbar gelagert ist.

Um eine kontrollierte Abwinkelung des Werkzeugträgers relativ zu dem Gelenkteil zu gewährleisten, ist der Werkzeugträger bevorzugt mit dem Schaft über mindestens eine Wälzkörperpaarung gekoppelt. So ist vorzugsweise vorgesehen, dass an dem distalen Ende des Schaftes mindestens ein starr mit dem Schaft verbundener Wälzkörper angeordnet ist, der in reibschlüssiger oder vorzugsweise in formschlüssiger Verbindung mit einem an dem Werkzeugträger starr angeordneten Wälzkörper steht. Bei einer über den Verzahnkörper eingeleiteten Abwinkelung des Gelenkteils führt dies zu einer definierten Abrollbewegung des werkzeugträgerseitigen Wälzkörpers auf dem schaftseitigen Wälzkörper und damit einhergehend zu einer definierten Abwinkelung des Instrumentenkopfes relativ zu dem Schaft.

Die Wälzkörper der Wälzkörperpaarung sind bevorzugt verzahnt ausgebildet. Vorteilhafterweise ist somit zumindest ein mit dem Schaft starr verbundenes Zahnradsegment vorgesehen, das mit einem mit dem Werkzeugträger starr verbundenen Zahnradsegment in Eingriff ist. Sowohl die Art der an den Zahnradsegmenten ausgebildeten Verzahnung als auch die Art der an dem Gelenkteil und dem Verzahnkörper ausgebildeten Verzahnungen ist grundsätzlich beliebig. Beispielsweise können die Verzahnungen als Evolventenverzahnung ausgebildet sein. Eine solche Verzahnung hat den Vorteil, dass sie vergleichsweise unempfindlich auf Abstandsänderungen der beiden Zahnradsegmente voneinander reagiert und ihre Herstellung vergleichsweise wenig aufwändig ist. Allerdings erfolgt die Abwälzbewegung der Zahnradsegmente bei einer Evolventenverzahnung nicht völlig gleitfrei, sodass es zu unerwünschten Stick-Slip-Effekten kommen kann. Zwar ist es generell möglich, diesen Stick-Slip-Effekten mit einer Fett- oder Ölschmierung der Verzahnung entgegenzuwirken, allerdings ist eine solche Maßnahme bei einem medizinischen Instrument nur bedingt oder gar nicht möglich. Daher ist es insbesondere dann, wenn es sich bei dem erfindungsgemäßen Instrument um ein medizinisch-endoskopisches Instrument handelt, zweckmäßiger, eine auf reine Rollreibung optimierte Verzahnung, beispielsweise eine Zykloidenverzahnung zu verwenden.

Das Übersetzungsverhältnis zwischen dem Verzahnkörper und dem verzahnten Bereich des Gelenkteils und das Übersetzungsverhältnis der Wälzkörperpaarung zwischen Werkzeugträger und Schaft sind zweckmäßigerweise so gewählt, dass ein möglichst großes Moment zum Abwinkeln des Instrumentenkopfes zur Verfügung steht.

Bevorzugt bilden der Verzahnkörper und die damit in Eingriff befindliche Verzahnung des Gelenkteils ein Untersetzungsgetriebe, das heißt, vorzugsweise weist der Verzahnkörper einen geringeren Wälzdurchmesser, als der an dem Gelenkteil ausgebildete verzahnte Bereich auf, sodass bei Drehung des Verzahnkörpers der Winkel, um den das Gelenkteil dann verschwenkt wird, kleiner als der Drehwinkel des Verzahnkörpers ist. Dies ist insofern vorteilhaft, als sich auf diese Weise der Winkel, in dem der Instrumentenkopf relativ zu dem Schaft abgewinkelt werden soll, besonders genau einstellen lässt.

Alternativ oder zusätzlich zu einem von dem Verzahnkörper und der Verzahnung des Gelenkteils gebildeten Untersetzungsgetriebe können auch ein schaftseitig angeordneter Wälzkörper und ein werkzeugträgerseitig angeordneter Wälzkörper der Wälzkörperpaarung zwischen dem Werkzeugträger und dem Schaft ein Untersetzungsgetriebe bilden. Hierbei ist vorgesehen, dass der schaftseitige Wälzkörper einen geringeren Wälzdurchmesser als der werkzeugträgerseitige Wälzkörper aufweist. Auch mit dieser Maßnahme wird bewirkt, dass der Winkel, in dem der Instrumentenkopf relativ zu dem Schaft abgewinkelt werden soll, besonders genau eingestellt werden kann.

Bevorzugt werden das von dem Verzahnkörper und der Verzahnung des Gelenkteils gebildete Untersetzungsgetriebe und das von den beiden Wälzkörpern der Wälzkörperpaarung zwischen Werkzeugträger und Schaft gebildete Untersetzungsgetriebe so ausgelegt, dass sich bei der Abwinkelung des Instrumentenkopfes das Moment um den Momentanpol der Bewegung gegenüber dem auf die Betätigungsrolle ausgeübten Moment vergrößert. Weiter bevorzugt werden das von dem Verzahnkörper und der Verzahnung des Gelenkteils gebildete Untersetzungsgetriebe und das von den beiden Wälzkörpern der Wälzkörperpaarung zwischen Werkzeugträger und Schaft gebildete Untersetzungsgetriebe so ausgelegt, dass die Betätigungsrolle und damit einhergehend der Verzahnkörper um nahezu +/- 180° verdreht werden müssen, um eine Abwinkelung des Werkzeugträgers relativ zu dem Schaft von +/- 120° zu bewirken. Zu diesem Zweck ist vorteilhaft vorgesehen, dass die Seilzüge des Seilzugpaares die Betätigungsrolle in einer Grundstellung des Instruments, in welcher der Instrumentenkopf in gerader Verlängerung des Schaftes angeordnet ist, jeweils in einem Winkelbereich von zumindest 180° umschlingen. Ist eine andere Abwinkelung des Werkzeugträgers als die vorab beschriebene von +/- 120° gefordert, so kann die Gesamtuntersetzung der beschriebenen Wälzkörperpaarungen konstruktiv so ausgeführt werden, dass die maximal mögliche Seilbewegung die geforderte Abwinkelung des Werkzeugträgers ergibt.

Nachfolgend ist die Erfindung anhand von in der Zeichnung dargestellten Ausführungsbeispielen näher erläutert. In der Zeichnung zeigt schematisch vereinfacht und in unterschiedlichen Maßstäben:
- Fig. 1: das distale Ende eines Instruments in perspektivischer Darstellung,
- Fig. 2: das Instrument nach Fig. 1 in einem Längsschnitt,
- Fig. 3: in einer vereinfachten Prinzipdarstellung die Abwinkelungsmechanik zwischen dem Schaft und dem Werkzeugträger sowie zwischen dem Verzahnkörper und dem Gelenkteil bei nicht abgewinkeltem Instrumentenkopf,
- Fig. 4: in einer vereinfachten Prinzipdarstellung die Abwinkelungsmechanik zwischen dem Schaft und dem Werkzeugträger sowie zwischen dem Verzahnkörper und dem Gelenkteil bei abgewinkeltem Instrumentenkopf und
- Fig. 5: in perspektivischer Darstellung einen zweigeteilt ausgebildeten Verzahnkörper.

Bei dem in der Zeichnung dargestellten Instrument handelt es sich um ein medizinisch-endoskopisches Instrument in Form einer Zange. Dieses Instrument weist einen länglichen, hohlzylindrisch ausgebildeten Schaft 2 auf, wobei in der Zeichnung aus Gründen der besseren Übersichtlichkeit lediglich das distale Ende des Schaftes 2 dargestellt ist. Auch die Steuerungseinrichtungen bzw. Antriebe am proximalen Ende des Schaftes 2 sind nicht dargestellt, da diese in bekannter Weise ausgebildet sein können.

Das distale Ende des Schaftes 2 wird von einem Endstück 4 gebildet. An das Endstück 4 schließt sich distalseitig ein Instrumentenkopf 6 an. Das hülsenförmig ausgebildete Endstück 4 weist an seinem distalen Ende zwei einander diametral gegenüberliegend angeordnete Vorsprünge 8 auf, die in Längsverlängerung des Schaftes 2 vorstehen. Zwischen den beiden Vorsprüngen 8 ist über einen Gelenkstift 10, der durch die Vorsprünge 8 geführt ist, ein längliches Gelenkteil 12 angelenkt. Das Gelenkteil 12 ist Teil des Instrumentenkopfes 6.

Distalseitig schließt sich an das Gelenkteil 12 ein Werkzeugträger 14 des Instrumentenkopfes 6 an. Korrespondierend zu den beiden an dem Endstück 4 ausgebildeten Vorsprüngen 8 sind an dem proximalen Ende des Werkzeugträgers 14 zwei einander diametral gegenüberliegend angeordnete Vorsprünge 16 ausgebildet, die sich in proximale Richtung erstrecken. Im Bereich der Vorsprünge 16 ist das Gelenkteil 12 mit dem Werkzeugträger 14 über einen Gelenkstift 18, der durch die Vorsprünge 16 geführt ist, schwenkbeweglich verbunden, wobei das Gelenkteil 12 in einen Zwischenraum zwischen den Vorsprüngen 16 eingreift.

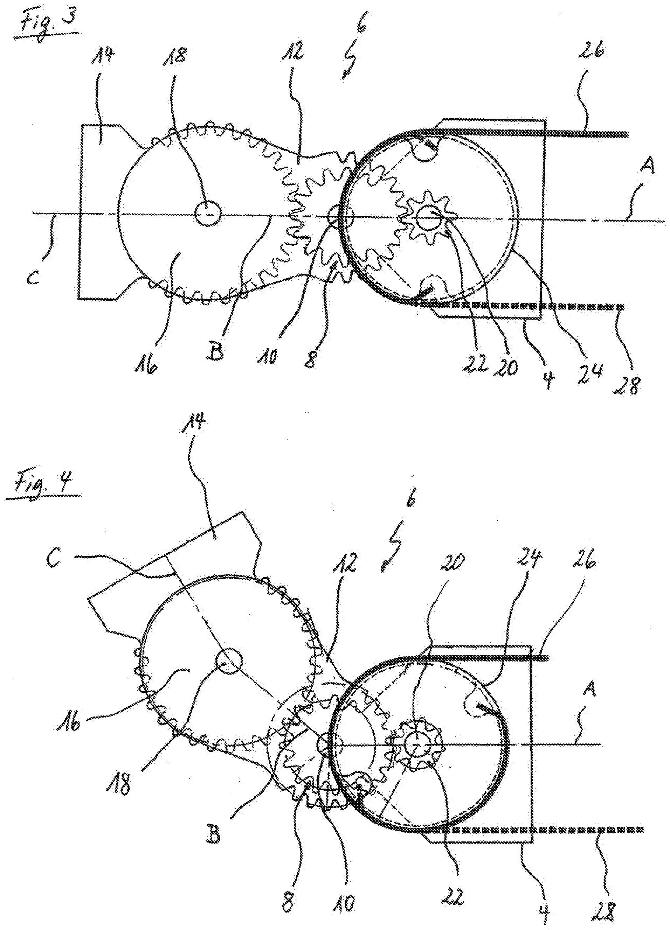

Aufgrund der schwenkbeweglichen Anordnung des Gelenkteils 12 an den Vorsprüngen 8 des Endstücks 4 und der schwenkbeweglichen Anordnung des Werkzeugträgers 14 an dem Gelenkteil 12 ist der Werkzeugträger 14 relativ zu dem Schaft ausgehend von einer Stellung, in der der Instrumentenkopf 6 in direkter Längsverlängerung des Schaftes 2 ausgerichtet ist und eine Mittelachse A des Schaftes 2 mit einer Mittelachse B des Gelenkteils 12 und einer Mittelachse C des Werkzeugträgers 14 in einer gemeinsamen Ebene liegen (Fig. 3), um einen Winkel verschwenkbar, der sich aus einem Schwenkwinkel des Gelenkteils 12 relativ zu der Mittelachse A des Schaftes 2 und einem Schwenkwinkel des Werkzeugträgers 14 relativ zu der Mittelachse B des Gelenkteils 12 zusammensetzt. Der gesamte Schwenkwinkel liegt hierbei in einem Winkelbereich von +/- 120°.

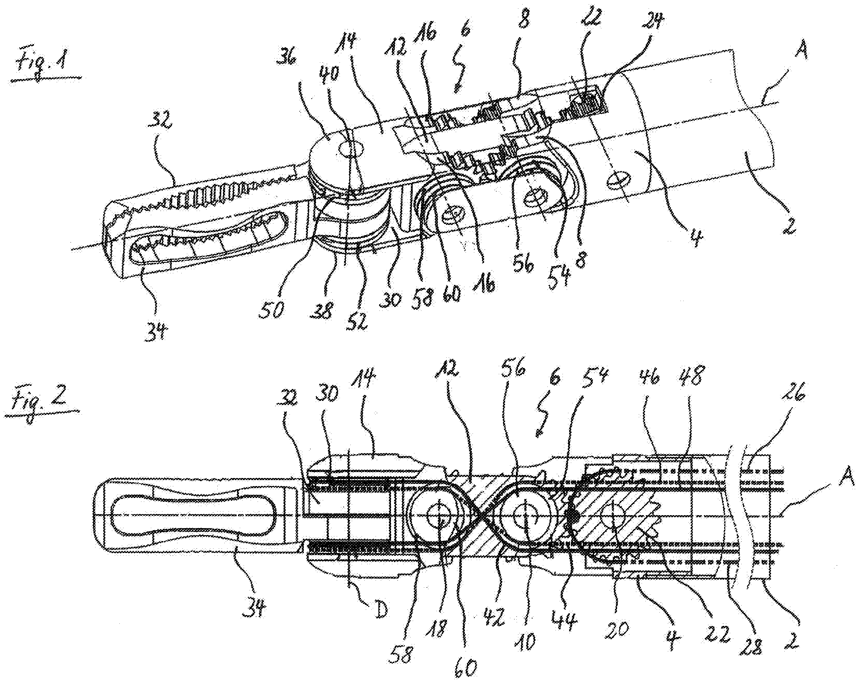

Um eine definierte Abwinkelung des Werkzeugträgers 14 relativ zu dem Schaft 2 zu ermöglichen, sind das Endstück 4 und der Werkzeugträger 14 über eine Wälzkörperpaarung miteinander verbunden. Zur Bildung dieser Wälzkörperpaarung weisen die distalen Enden der an dem Endstück 4 ausgebildeten Vorsprünge 8 und die proximalen Enden der an dem Werkzeugträger 14 ausgebildeten Vorsprünge 16 jeweils einen Verzahnabschnitt in Form eines Zahnradsegments auf, wobei die an den Vorsprüngen 8 und 16 ausgebildeten Verzahnabschnitte miteinander in Eingriff sind. Die Verzahnungen der Verzahnabschnitte der Vorsprünge 8 und 16 sind als Zykloidenverzahnung ausgebildet. Bei dem in den Fig. 1 und 2 dargestellten Ausführungsbeispiel weisen die an den Vorsprüngen 8 und 16 ausgebildeten Verzahnabschnitte den gleichen Wälzkreisdurchmesser auf, während in der Darstellung nach den Fig. 3 und 4 der Wälzkreisdurchmesser der an den Vorsprüngen 8 ausgebildeten Verzahnabschnitte nur halb so groß, wie derjenige, der an den Vorsprüngen 16 ausgebildeten Verzahnabschnitte ist, sodass die an den Vorsprüngen 8 und 16 ausgebildeten Verzahnabschnitte ein Untersetzungsgetriebe bilden.

An dem abgerundeten proximalen Ende des Gelenkteils 12 ist eine zahnradsegmentförmige Verzahnung ausgebildet, die mit einem proximalseitig des Gelenkteils 12 in dem Endstück 4 an einem Gelenkstift 20 drehbar gelagerten Verzahnkörper 22 in Eingriff ist. Auch die an dem Verzahnkörper 22 ausgebildete Verzahnung sowie die an dem proximalen Ende des Gelenkteils 12 ausgebildete Verzahnung sind als Zykloidenverzahnung ausgebildet. Bei dem in den Fig. 1 und 2 dargestellten Ausführungsbeispiel sind der Wälzkreisdurchmesser der an dem Gelenkteil 12 ausgebildeten Verzahnung und der Wälzkreisdurchmesser des Verzahnkörpers 22 identisch, während in der Darstellung nach den Fig. 3 und 4 der Wälzkreisdurchmesser des Verzahnkörpers 22 nur halb so groß wie derjenige, der an dem Gelenkteil 12 ausgebildeten Verzahnung ist, sodass der Verzahnkörper 22 mit der an dem Gelenkteil 12 ausgebildeten Verzahnung ein Untersetzungsgetriebe bildet.

Auf dem Gelenkstift 20 ist auch eine mit dem Verzahnkörper 22 drehfest verbundene Betätigungsrolle 24 drehbar gelagert. Diese Betätigungsrolle 24 weist einen Außendurchmesser auf, der im Wesentlichen dem Innendurchmesser des Schaftes 2 entspricht. An der Betätigungsrolle 24 ist ein Seilzugpaar mit Seilzügen 26 und 28 befestigt (Fig. 3 und 4). Die Befestigung der beiden Seilzüge 26 und 28 an der Betätigungsrolle 24 ist derart, dass die Seilzüge 26 und 28 die Betätigungsrolle 24 in einer Grundstellung des Instruments, in welcher der Instrumentenkopf 6 in gerader Verlängerung des Schaftes 2 angeordnet ist, jeweils in einem Winkelbereich von 180° umschlingen (Fig. 3). Ausgehend von der Betätigungsrolle 24 sind die Seilzüge 26 und 28 zu dem proximalen Ende des Schaftes 2 geführt. Dort, proximalseitig des Schafts 2 sind die Seilzüge 26 und 28 mit einer in der Zeichnung nicht dargestellten Steuerungseinrichtung verbunden, bei der es sich je nach Art des Instrumentes um eine manuell zu betätigende Handhabe oder um eine Steuerungsschnittstelle eines robotischen Operationssystems handeln kann. Mit der Steuerungseinrichtung wird die Abwinkelung des Instrumentenkopfes 6 gesteuert.

Der Werkzeugträger 14 weist distalseitig einen zum distalen Ende des Werkzeugträgers 14 hin offenen Einschnitt 30 auf. In dem Einschnitt 30 sind zwei Maulteile 32 und 34 eines Maulwerkzeugs angelenkt. Die Anlenkung der Maulteile 32 und 34 in dem Einschnitt 30 erfolgt über einen in der Zeichnung nicht dargestellten Gelenkstift, der durch an zwei den Einschnitt 30 begrenzenden Wandungsabschnitten 36 und 38 ausgebildete Löcher 40 geführt ist. Der Gelenkstift bildet eine Schwenkachse D, um die die Maulteile 32 und 34 in einer Ebene normal zur Abwinkelungsebene des Instrumentenkopfes 6 verschwenkbar sind.

Zur Steuerung der Maulteile 32 und 34 dienen Seilzüge 42, 44, 46 und 48 (Fig. 2). Distalseitig sind die Seilzüge 42 und 44 an einer Betätigungsrolle 50 befestigt, die drehfest mit dem Maulteil 32 verbunden ist und um die Schwenkachse D der Maulteile 32 und 34 drehbar ist. Korrespondierend hierzu sind die Seilzüge 46 und 48 an einer um die Schwenkachse D drehbaren Betätigungsrolle 52 befestigt, die drehfest mit dem Maulteil 34 verbunden ist.

Außenseitig der an dem Endstück 4 ausgebildeten Vorsprünge 8 ist jeweils eine Führungsrolle 54 auf dem Gelenkstift 10 drehbar gelagert. Zudem ist eine weitere Führungsrolle 56 jeweils außenseitig der Führungsrolle 54 auf dem Gelenkstift 10 gelagert. Auch an dem Gelenkstift 18 sind außenseitig der an dem Werkzeugträger 14 ausgebildeten Vorsprünge 16 jeweils eine Führungsrolle 58 und außenseitig der Führungsrollen 58 jeweils eine Führungsrolle 60 drehbar gelagert. Die in Fig. 1 sichtbaren Führungsrollen 54, 56, 58 und 60 befinden sich nochmal in an der Mittelachse B gespiegelter Anordnung nicht sichtbar im hinteren Bereich des Instrumentenkopfs 6. Im Bereich der Abwinkelung des Instrumentenkopfes 6 dienen die Führungsrollen 54 und 58 zur Führung der durch den Schaft 2 nach proximalseitig des Schaftes 2 geführten Seilzüge 42 und (nicht sichtbar im hinteren Bereich) 46, während die Führungsrollen 56 und 60 zur Führung der durch den Schaft 2 nach proximalseitig des Schaftes 2 geführten Seilzüge (nicht sichtbar im hinteren Bereich) 44 und 48 dienen. Die Führung des Seilzugs 42 durch den Instrumentenkopf 6 erfolgt durch die Führungsrollen 54 und 58. Die Führung des Seilzugs 48 erfolgt durch die Führungsrollen 56 und 60. Dabei überkreuzen sich die Seilzüge 42 und 48 in einem Zwischenraum zwischen den Führungsrollen 54, 56, 58 und 60 ohne sich zu berühren. In gleicher Weise kreuzen sich die Seilzüge 44 und 46 im hinteren nicht sichtbaren Bereich zwischen den Führungsrollen ohne sich zu berühren. Proximalseitig des Schaftes 2 sind die Seilzüge 42, 44, 46 und 48 zur Steuerung der Maulteile 32 und 34 mit einer Steuerungseinrichtung bewegungsgekoppelt, bei der es sich um eine Handhabe oder eine Steuerungsschnittstelle eines robotischen Operationssystems handeln kann, je nachdem ob das Instrument eine manuell zu bedienendes Instrument ist oder ein Teil eines robotischen Operationssystems ist.

Anhand der Fig. 3 und 4 ist nachfolgend die Steuerung der Abwinkelung des Instrumentenkopfes 6 näher erläutert. Ausgehend von der in Fig. 3 dargestellten Stellung, in welcher der Instrumentenkopf 6 in direkter Längsverlängerung des Schaftes 2 ausgerichtet ist, wird der Seilzug 28 auf Zug beansprucht. Hierdurch wird die Betätigungsrolle 24 und der damit drehfest verbundene Verzahnkörper 22 entgegen dem Uhrzeigersinn gedreht, was wiederum eine Schwenkbewegung des Gelenkteils 12 im Uhrzeigersinn zur Folge hat (Fig. 4). Während der Schwenkbewegung des Gelenkteils 12 wälzen die Verzahnungen der an den Vorsprüngen 16 des Werkzeugträgers 14 ausgebildeten Verzahnabschnitte auf den an den Vorsprüngen 8 am Endteil 4 ausgebildeten Verzahnabschnitten ab, sodass sich der Schwenkbewegung des Gelenkteils 12 eine Schwenkbewegung des Werkzeugträgers 14 überlagert. Hierbei bewirken die gewählten Übersetzungsverhältnisse bei den Wälzkörperpaarungen der Vorsprünge 8 mit den Vorsprüngen 16 und bei der Wälzkörperpaarung des Verzahnkörpers 22 mit dem Gelenkteil 12, dass sich das Moment um den Momentanpol der Bewegung gegenüber dem auf die Betätigungsrolle 24 ausgeübten Moment vergrößert und die Betätigungsrolle 24 nahezu um 180° verdreht werden muss, damit sich eine Gesamtabwinkelung des Instrumentenkopfes 6 von 120° ergibt.

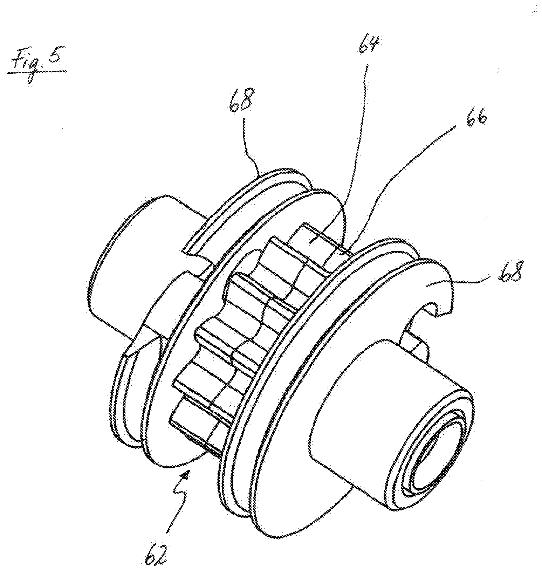

In den Figuren 1 bis 4 ist der grundsätzliche Aufbau des Instrumentes dargestellt, wie ein Verzahnkörper 22 gemäß der Erfindung aussieht ist in Fig. 5 anhand des Verzahnkörpers 62 dargestellt. Dieser Verzahnkörper 62 ist quer zu seiner Drehachse zweigeteilt ausgebildet und weist zwei Teile 64 und 66 auf. Die beiden Teile 64 und 66 des Verzahnkörpers sind relativ zueinander verdrehbar. An den voneinander abgewandten Stirnseiten der Teile 64 und 66 ist mit diesen jeweils eine Betätigungsrolle 68 drehfest verbunden. Zur Betätigung des Verzahnkörpers 62 ist mit jeder der Betätigungsrollen ein in der Zeichnung nicht dargestellter vorgespannter Seilzug verbunden. Die Befestigung der Seilzüge an den Betätigungsrollen 68 ist derart, dass sie antagonistisch auf den Verzahnkörper 62 wirken.

### Bezugszeichenliste

- 2: Schaft
- 4: Endstück
- 6: Instrumentenkopf
- 8: Vorsprung
- 10: Gelenkstift
- 12: Gelenkteil
- 14: Werkzeugträger
- 16: Vorsprung
- 18: Gelenkstift
- 20: Gelenkstift
- 22: Verzahnkörper
- 24: Betätigungsrolle
- 26: Seilzug
- 28: Seilzug
- 30: Einschnitt
- 32: Maulteil
- 34: Maulteil
- 36: Wandungsabschnitt
- 38: Wandungsabschnitt
- 40: Loch
- 42: Seilzug
- 44: Seilzug
- 46: Seilzug
- 48: Seilzug
- 50: Betätigungsrolle
- 52: Betätigungsrolle
- 54: Führungsrolle
- 56: Führungsrolle
- 58: Führungsrolle
- 60: Führungsrolle
- 62: Verzahnkörper
- 64: Teil
- 66: Teil
- 68: Betätigungsrolle

- A: Mittelachse
- B: Mittelachse
- C: Mittelachse
- D: Schwenkachse

## Patentansprüche

1. Schaftinstrument, insbesondere medizinisch-endoskopisches Schaftinstrument oder Technoskop, mit einem Schaft (2) und mit einem Instrumentenkopf (6), welcher mindestens ein an dem distalen Schaftende abwinkelbar angelenktes Gelenkteil (12) und einen distalseitig an dem Gelenkteil (12) in der Abwinkelungsebene des Gelenkteils (12) abwinkelbar angelenkten Werkzeugträger (14) aufweist, wobei an dem proximalen Ende des Gelenkteils (12) eine Verzahnung ausgebildet ist, welche mit einem in dem Schaft (2) drehbar gelagerten und mit einem Seilzugpaar zur Bewegungssteuerung des Instrumentenkopfes (6) bewegungsgekoppelten Verzahnkörper (62) in Eingriff ist, **dadurch gekennzeichnet, dass** der Verzahnkörper (62) quer zu seiner Drehachse zweigeteilt ist, wobei die beiden Teile (64, 66) des Verzahnkörpers (62) relativ zueinander verdrehbar sind und jeder der beiden Teile (64, 66) des Verzahnkörpers (62) drehfest mit einer Betätigungsrolle (68) verbunden ist, an welcher jeweils ein Seilzug bezogen auf den anderen Seilzug antagonistisch angreift.

2. Schaftinstrument nach Anspruch 1, bei dem das Seilzugpaar mit zwei Betätigungsrollen (68) verbunden ist, welche achsgleich mit dem Verzahnkörper (62) in dem Schaft (2) drehbar gelagert sind und mit dem Verzahnkörper (22, 62) jeweils drehfest verbunden sind.

3. Schaftinstrument nach Anspruch 2, bei dem die Betätigungsrolle (24) einen Durchmesser aufweist, welcher im Wesentlichen mit dem Innendurchmesser des Schaftes (2) übereinstimmt.

4. Schaftinstrument nach einem der vorangehenden Ansprüche, bei dem der Verzahnkörper (62) und der an dem proximalen Ende des Gelenkteils (12) ausgebildete Verzahnabschnitt eine Zykloidenverzahnung aufweisen.

5. Schaftinstrument nach einem der vorangehenden Ansprüche, bei dem der Werkzeugträger (14) mit dem Schaft (2) über mindestens eine Wälzkörperpaarung gekoppelt ist.

6. Schaftinstrument nach Anspruch 5, bei dem die Wälzkörper der Wälzkörperpaarung verzahnt ausgebildet sind.

7. Schaftinstrument nach einem der vorangehenden Ansprüche, bei dem der Verzahnkörper und die damit in Eingriff befindliche Verzahnung des Gelenkteils ein Untersetzungsgetriebe bilden.

8. Schaftinstrument nach einem der Ansprüche 5 bis 7, bei dem ein schaftseitig angeordneter Wälzkörper und ein werkzeugträgerseitig angeordneter Wälzkörper ein Untersetzungsgetriebe bilden.

9. Schaftinstrument nach einem der Ansprüche 2 bis 8, bei dem die Seilzüge (26, 28) des Seilzugspaares die Betätigungsrollen (68) in einer Grundstellung des Instruments, in welcher der Instrumentenkopf (6) in gerader Verlängerung des Schaftes (2) angeordnet ist, jeweils in einem Winkelbereich von zumindest 180° umschlingen.

## Claims

1. A shank instrument, in particular a medical endoscopic shank instrument or technoscope, with a shank (2) and with an instrument head (6), said instrument head comprising at least one joint part (12) articulated on the distal shank end in an angularly bendable manner and a tool carrier (14) which at the distal side is articulated on the joint part (12) in an angularly bendable manner in the angular bending plane of the joint part (12), wherein a toothing which is meshed with a toothed body (62) rotatably mounted in the shank (2) and coupled in movement to a pull cable pair for the movement control of the instrument head (6) is formed at the proximal end of the joint part (12) **characterised in that** the toothed body (62) is divided in two transversely to its rotation axis, wherein the two parts (64, 66) of the toothed body (62) are rotatable relative to one another and each of the two parts (64, 66) of the toothed body (62) is connected in a rotationally fixed manner to an actuation roller (68), on which actuation roller a pull cable engages in each case, in an antagonistic manner with respect to the other pull cable.

2. A shank instrument according to claim 1, with which the pull cable pair is connected with two actuation rollers (68) which are rotatably mounted in the shank (2) in a manner having the same axis as the toothed body (62) and each are connected to the toothed body (22, 62) in a rotationally fixed manner.

3. A shank instrument according to claim 2, with which the actuation roller (24) has a diameter which corresponds essentially to the inner diameter of the shank (2).

4. A shank instrument according to one of the preceding claims, with which the toothed body (62) and the toothed section which is formed at the proximal end of the joint part (12) comprise a cycloidal toothing.

5. A shank instrument according to one of the preceding claims, with which the tool carrier (14) is coupled in movement to the shank (2) via at least one roller body pairing.

6. A shank instrument according to claim 5, with which the roller bodies of the roller body pairing are designed in a toothed manner.

7. A shank instrument according to one of the preceding claims, with which the toothed body and the toothing of the joint part which is meshed with this form a step-down gear.

8. A shank instrument according to one of the claims 5 to 7, with which a roller body which is arranged on the shank side and a roller body arranged on the tool carrier side form a step-down gear.

9. A shank instrument according to one of the claims 2 to 8, with which the pull cables (26, 28) of the pull cable pair wrap the actuation roller (68) in each case in an angular range of at least 180°, in a basic position of the instrument, in which the instrument head (6) is arranged in the straight extension of the shank (2).

## Revendications

1. Instrument à tige, en particulier instrument à tige endoscopique médical ou endoscope technique, comprenant une tige (2) et une tête à instrument (6) comportant au moins une partie d'articulation (12) articulée à l'extrémité distale de tige de façon à pouvoir être inclinée et un porte-outil (14) articulé du côté distal à la partie d'articulation (12) de façon à pouvoir être incliné dans le plan d'inclinaison de la partie d'articulation (12), une denture, qui est en prise avec un corps denté (62) monté de manière rotative dans la tige (2) et à couplage de mouvement grâce à une paire de câbles afin de commander le mouvement de la tête à instrument (6), étant formée à l'extrémité proximale de la partie d'articulation (12), **caractérisé en ce que** le corps denté (62) est divisé en deux parties transversalement par rapport à son axe de rotation, les deux parties (64, 66) du corps denté (62) étant rotatives l'une par rapport à l'autre et chacune des deux parties (64, 66) du corps denté (62) étant reliée de manière solidaire en rotation à un rouleau d'actionnement (68) auquel un câble respectif vient en prise de manière antagoniste par rapport à l'autre câble.

2. Instrument à tige selon la revendication 1, dans lequel la paire de câbles est reliée à deux rouleaux d'actionnement (68) qui sont montés de manière rotative dans la tige (2) et de manière coaxiale par rapport au corps denté (62) et qui sont respectivement reliés de manière solidaire en rotation au corps denté (22, 62).

3. Instrument à tige selon la revendication 2, dans lequel le rouleau d'actionnement (24) présente un diamètre qui coïncide essentiellement avec le diamètre intérieur de la tige (2).

4. Instrument à tige selon l'une quelconque des revendications précédentes, dans lequel le corps denté (62) et la section dentée formée à l'extrémité proximale de la partie d'articulation (12) présentent une denture cycloïdale.

5. Instrument à tige selon l'une quelconque des revendications précédentes, dans lequel le porte-outil (14) est couplé à la tige (2) par l'intermédiaire d'au moins un appariement d'organes de roulement.

6. Instrument à tige selon la revendication 5, dans lequel les organes de roulement de l'appariement d'organes de roulement sont réalisés dentés.

7. Instrument à tige selon l'une quelconque des revendications précédentes, dans lequel le corps denté et la denture de la partie d'articulation étant en prise avec celui-ci forment un engrenage réducteur.

8. Instrument à tige selon l'une quelconque des revendications 5 à 7, dans lequel un organe de roulement agencé côté tige et un organe de roulement agencé côté porte-outil forment un engrenage réducteur.

9. Instrument à tige selon l'une quelconque des revendications 2 à 8, dans lequel, dans une position initiale de l'instrument au sein de laquelle la tête à instrument (6) est agencée dans le prolongement rectiligne de la tige (2), les câbles (26, 28) de la paire de câbles entourent les rouleaux d'actionnement (68) respectivement dans une plage angulaire d'au moins 180°.
